# EUROPÄISCHE PATENTSCHRIFT

(11) **EP 3 013 429 B1**
(45) Veröffentlichungstag und Bekanntmachung des Hinweises auf die Patenterteilung: **02.01.2019**
(21) Anmeldenummer: 14735850.1
(22) Anmeldetag: 25.06.2014
(51) Int. Cl.: A61Q 19/10, A61Q 5/02, A61K 8/42, C11D 1/52, A61Q 5/00, A61K 8/41, C11D 3/32, C11D 1/90, C11D 1/14, C11D 1/65, A61K 8/46

(54) **VERWENDUNG VON SPEZIELLEN N-METHYL-N-ACYLGLUCAMINEN IN HANDGESCHIRRSPÜLMITTELN**
USE OF SPECIAL N-METHYL-N-ACYLGLUCAMINES IN HAND DISHWASHING AGENTS
UTILISATION DE N-METHYL-N-ACYLGLUCAMINES SPÉCIALES DANS DES DÉTERGENTS POUR VAISSELLE À LA MAIN

(30) Priorität: 28.06.2013 DE 102013212738; 29.11.2013 DE 102013224559
(43) Veröffentlichungstag der Anmeldung: 04.05.2016
(60) Teilanmeldung: 17169355.9
(73) Patentinhaber: Clariant International Ltd, 4132 Muttenz (CH)
(72) Erfinder: KLUG, Peter, 63762 Grossostheim (DE); MILDNER, Carina, 65366 Geisenheim (DE); NEUHOFF, Henrike, 65193 Wiesbaden (DE)
(74) Vertreter: Paczkowski, Marcus
(86) Internationale Anmeldenummer: PCT/EP2014/001722
(87) Internationale Veröffentlichungsnummer: WO 2014/206554

(56) Entgegenhaltungen:
- EP-A1- 0 285 768
- EP-A2- 1 043 017
- EP-A2- 1 110 944
- WO-A1-2014/170025
- WO-A2-2013/178668
- WO-A2-2013/178670
- WO-A2-2013/178671
- WO-A2-2013/178679
- WO-A2-2013/178697
- WO-A2-2013/178701
- DE-A1- 19 507 531
- DE-C1- 4 435 383
- DE-U1-202013 011 412
- DE-U1-202013 011 413
- US-A1- 2009 023 622

## Beschreibung

Offenbart wird die Verwendung von speziellen N-Alkyl-N-acylglucaminen in Handgeschirrspülmitteln sowie Handgeschirrspülmittel enthaltend diese N-Alkyl-N-acylglucamine.
An kosmetische Reinigungsprodukte für die Haut werden hohe Anforderungen gestellt. Sie sollen ein gutes Erscheinungsbild zeigen, toxikologisch und ökotoxikologisch unbedenklich sein, exzellent reinigen und dennoch ein angenehmes Hautgefühl erzeugen. Analoge Anforderungen werden auch an Handgeschirrspülmittel gestellt.
Die in solchen Produkten enthaltenen Tensidsysteme müssen hierbei eine Doppelfunktion erfüllen. Einerseits soll eine ausreichende Reinigungswirkung vorhanden sein, die aber oft bei zu starker Entfettung in ein stumpfes Hautgefühl umschlägt. Andererseits soll nach der Anwendung ein gepflegtes Hautgefühl gegeben sein.
Diese Effekte erreicht man normalerweise, in dem man dem zur Reinigung vorhandenen Tensidsystem zusätzliche hautpflegende Stoffe zusetzt.
Dies können z. B. kationische Tenside (Hydroxyethylquats), kationische Polymere (Polyquaternium-7, Polyquaternium-10) oder kationische Guarderivate, Chitosanderivate) sein. Weiterhin werden hierzu Fettsäureester (Glyceryloleat) oder ethoxylierte Fettsäureester (PEG-7 Glycerylcocoat) verwendet. All diese Substanzen weisen jedoch Nachteile bei ihrer Anwendung auf, insbesondere die kationischen Komponenten sind aufgrund ihrer kationischen Natur nur begrenzt mit den üblicherweise eingesetzten Tensidsystemen oder anderen Additiven wie z.B. Trübungsmitteln kompatibel.
In der EP-A 1 043 017 (DE 199 16 090) sind Hautpflegemittel wie Cremes, Pflegelotionen und Sonnenschutzmittel offenbart, die N-Acyl-N-alkylglucamide enthalten. Die N-Acyl-N-alkylglucamide sollen die Funktion natürlicher Ceramide übernehmen und einen Beitrag zur Wiederherstellung einer gestörten Haut-Barriere-Funktion leisten. Eine Beeinflussung des Hautgefühls durch solche Stoffe ist jedoch nicht beschrieben.

Es wurde gefunden, dass bestimmte N-Methyl-N-acylglucamine, wie sie teilweise in der EP-A 1 043 017 beschrieben sind, bei Verwendung in Tensidsystemen zur Hautreinigung nicht nur gute Reinigungseigenschaften aufweisen, sondern auch zu einer Verbesserung des Hautgefühls nach der Reinigung beitragen. Es war überraschend, dass somit ein Tensid hautpflegende Aufgaben in dem Pflegemittel übernehmen kann, für die üblicherweise weitere Additive zugesetzt werden müssen. EP-A 0 285 768 befasst sich mit der Verwendung von N-Polyhydroxyalkylfettsäureamiden als Verdickungsmittel für flüssige wässrige Tensidsysteme. EP-A 1 110 944 betrifft ein Verfahren zur Herstellung von Fettsäure-N-alkylpolyhydroxyamiden und die Verwendung eines solche enthaltenen Produktgemisches als Tensid oder Emulgator in Wasch- und Reinigungsmitteln sowie in kosmetischen Zubereitungen, in Pflanzenschutzformulierungen und in Zubereitungen für die Metallbearbeitung. In US-A 2009/023622 werden wässrige oberflächenaktive Formulierungen, die Polypropylenglykol(3)myristylether enthalten, gelehrt. DE-A 195 07 531 betrifft kosmetische und pharmazeutische Zubereitungen, die als O/W-Emulgatoren Fettsäure-N-alkylpolyhydroxyalkylamide enthalten. In DE-A 44 35 383 werden kosmetische Mittel, die Fettsäure-N-alkylglucamide und Siliconverbindungen enthalten, gelehrt.

Gegenstand der Erfindung ist daher die Verwendung von N-Alkyl-N-acylglucaminen der Formel (I) in Handgeschirrspülmitteln, insbesondere als hautpflegende Komponente, die ein wässriges Tensidsystem, enthaltend mindestens ein anionisches Tensid, aufweisen, 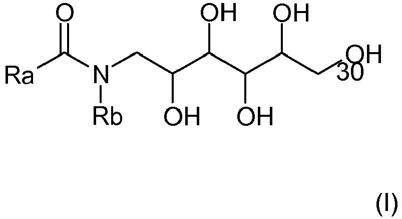 wobei in der Formel (I)
Ra einen linearen oder verzweigten, gesättigten oder ungesättigten C₅-C₂₁-Alkylrest, vorzugsweise C₁₁-C₁₇-Alkylrest, und
Rb einen C₁-C₄ Alkylrest, vorzugsweise Methyl, bedeutet, und
wobei die N-Alkyl-N-acylglucamine (I) mindestens 8 Gew.-%, bezogen auf die Gesamtmenge an N-Alkyl-N-acylglucaminen (I), Verbindungen mit einem ein oder mehrfach ungesättigten C₁₈-Fettsäurerest Ra-CO- enthalten.
Weiterhin Gegenstand der Erfindung ist ein Handgeschirrspülmittel, enthaltend
(a) ein oder mehrere N-Methyl-N-acylglucamine (I), die mindestens 8 Gew.-%, bezogen auf die Gesamtmenge an N-Alkyl-N-acylglucaminen (I), Verbindungen mit einem gesättigten C16-, C18- oder einem ein oder mehrfach ungesättigten C18-Fettsäurerest Ra-CO- enthalten als Komponente (A),
(b) ein oder mehrere anionische Tenside aus der Gruppe der Alkylethersulfate, Alkylsulfate und N-Acyl-aminosäuretenside als Komponente (B),
(c) gegebenenfalls ein oder mehrere Betain-Tenside als Komponente (C),
(d) gegebenenfalls ein oder mehrere weitere Tenside als Komponente (D),
(e) ein oder mehrere Rückfettungsmittel als Komponente (E),
(f) Wasser als Komponente (F) sowie
(g) gegebenenfalls weitere Additive, wie Konservierungsmittel, Duftstoffe und Farbstoffe, als Komponente (G).

Die erfindungsgemäß verwendeten Tensidsysteme, enthaltend langkettige, besonders ungesättigte Glucamide erzeugen neben der Reinigungs- auch eine hautpflegende Wirkung, die den Einsatz zusätzlicher Additive, wie weiterer Fettsäureester, ethoxylierter Fettsäureester oder kationischer Substanzen überflüssig machen oder reduzieren kann und dementsprechend zu einer Vereinfachung der Gesamtrezeptur beiträgt.
Die erfindungsgemäß verwendeten N-Alkyl-N-acylglucamine (I), auch als N-Alkyl-N-1-Desoxisorbityl-Fettsäureamide bekannt, enthalten mindestens 8, bevorzugt mindestens 10, besonders bevorzugt mindestens 15, ganz besonders bevorzugt mindestens 30 und insbesondere bevorzugt mindestens 60, Gew.-% N-Alkyl-N-acylglucamine (I) mit ein oder mehrfach ungesättigten C₁₈- Acylrest CO-Ra.
Bevorzugt sind N-Alkyl-N-acylglucamine (I), bei denen der Rest CO-Ra von Laurinsäure, Palmitinsäure, Stearinsäure, Ölsäure, Linolsäure oder Linolensäure abgeleitet ist. Insbesondere bevorzugt sind N-Methyl-N-acylglucamine der Formel (I), wobei der Acylrest CO-Ra abgeleitet ist von der Laurinsäure, der Ölsäure, der Linolsäure oder der Linolensäure.
Bevorzugt sind weiterhin N-Alkyl-N-acylglucamine (I), die mindestens 8 Gew.-%, N-Alkyl-N-acylglucamine (I) mit einem einfach ungesättigten C₁₈-Fettsäurerest enthalten.
Enthalten die erfindungsgemäß eingesetzten N-Alkyl-N-acylglucamine (I) sowohl ungesättigte langkettige als auch mittelkettige Fettsäurereste (C12), erhält man Tensidsysteme, die eine hervorragende Kombination von Wasserlöslichkeit, Reinigungsleistung, Schaumbildung und hautpflegenden Eigenschaften zeigen. Solche Fettsäuremischungen weisen zum Beispiel natürliches Palmkern- und Kokosöl auf.
Bevorzugt sind daher auch Ausführungsformen, bei denen die N-Alkyl-N-acylglucamine (I) mindestens 8 Gew.-% N-Alkyl-N-acylglucamine (I) mit einem ein oder mehrfach ungesättigten ungesättigten C₁₈-Fettsäurerest enthalten und mindestens 30 Gew.-% mit einem gesättigten C₁₂- Fettsäurerest enthalten.

Bevorzugt enthalten die in Handgeschirrspülmitteln verwendeten N-Methyl-N-acylglucamine nur geringe Anteile an mittleren Fettsäuren abgeleiteten N-Methyl-N-acylglucaminen, welche C₆-C₁₀-Acylgruppen enthalten. Bevorzugt beträgt der Anteil solcher mittellangen Fettsäuren nicht mehr als 15, besonders bevorzugt 10, insbesondere 5, Gew.-%.

Die N-Methyl-N-acylglucamine (I) können, wie in EP-A 0 550 637 B1 und EP-A 0 285 768 beschrieben, durch Umsetzung der entsprechenden Fettsäureester bzw. Fettsäureestergemische mit N-Methylglucamin in Gegenwart eines Hydroxylgruppen oder Alkoxylgruppen aufweisenden Lösungsmittels hergestellt werden. Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden. Geeignete Fettsäureester, die mit den N-Methylglucaminen zu erfindungsgemäßen Glucamiden umgesetzt werden, sind im Allgemeinen die Methylester, die durch Umesterung aus natürlichen Fetten und Ölen, beispielsweise den Triglyceride, gewonnen werden.

Als ungesättigte C₁₈-Acylgruppen im Sinne der Erfindung sind Fettsäurereste mit einer oder mehreren Doppelbindungen zu verstehen. Bevorzugt sind hierbei Reste, die sich von der Ölsäure, der Linol- und der Linolensäure ableiten.

Die erfindungsgemäß eingesetzten wässrigen Tensidsysteme enthalten ein oder mehrere anionische Tenside, bevorzugt aus der Gruppe der Alkylsulfate und Alkylethersulfate, besonders bevorzugt in Kombination mit Betainen.

In einer weiteren Ausführungsform enthalten die Tensidsysteme neben Alkylethersulfaten und/oder Alkylsulfaten Fettsäurealkanolamide.

Bevorzugte Alkylsulfate sind die C₈-C₂₀-Alkylsulfate, insbesondere die linearen C₈-C₂₀-Alkylsulfate in Form ihrer Natrium-, Kalium- oder Ammoniumsalze. Beispiele für Alkylsulfate sind Laurylsulfat, Cocosalkylsulfat und Talgalkylsulfat. Besonders bevorzugt ist Laurylsulfat.

Bevorzugte Alkylethersulfate sind die C₈-C₂₀-Alkylethersulfate, besonders bevorzugt sind die linearen C₈C₂₀-Alkylethersulfate, insbesondere die von den ethoxylierten Fettalkoholen abgeleiteten Alkylglykolethersulfate, in Form ihrer Natrium-, Kalium- oder Ammoniumsalze. Beispiele für Alkylethersulfate sind Laurylethersulfat, Cocosalkylethersulfat und Talgalkylethersulfat. Beispiele für Glykolethersulfate sind Lauryltriethylenglykolethersulfat, Cocosalkyltriethylenglykolethersulfat und Talgalkylhexaethylenglykolethersulfat. Insbesondere bevorzugt ist Laurylglykolethersulfat, beispielsweise, Lauryldiethylenglykolethersulfat oder Lauryltriethylenglykolethersulfat, speziell in Form der Natriumsalze.

In einer weiteren bevorzugten Ausführungsform der Erfindung enthalten die Tensidsysteme ein oder mehrere N-Acylaminosäuretenside als anionische Tenside. Im Rahmen einer bevorzugten Ausführungsform ist der Aminosäurerest solcher N-Acyl-aminosäuretenside ausgewählt aus der Gruppe bestehend aus proteinogenen Aminosäuren, deren N-alkylierten Derivaten oder Mischungen daraus.

Besonders bevorzugt als N-Acyl-aminosäuretenside sind Acylglycinate, Acylalaninate, Acylaspartate, Acylglutamate, Acylsarkosinate oder Mischungen davon. Ganz besonders bevorzugt sind die N-Acyl-aminosäuretenside ausgewählt aus der Gruppe bestehend aus Acylglycinat, Acylaspartat, Acylglutamat, Acylsarkosinat und Mischungen daraus.

Ganz besonders bevorzugt bestehen die N-Acyl-aminosäuretenside aus mindestens einer C₈-C₂₂-acylierten Aminosäure, insbesondere deren N-alkylierten Derivaten. Bevorzugt sind die entsprechenden Lauroyl - oder Cocoylderivate der Aminosäuren.

Insbesondere bevorzugt sind daher Natrium Cocoyl-Glycinat, Kaliumcocoylglycinat, Natriumlauroylglycinat, Kaliumlauroylglycinat, Natriumcocoylglutamat, Natriumlauroylglutamat, Natriumcocoylaspartat, Natriumlauroylaspartat und Natriumlauroylsarkosinat.

Vorzugsweise enthalten die wässrigen Tensidlösungen neben dem mindestens einen anionischen Tensid ein Betain-Tensid (C).

Betain-Tenside enthalten im selben Molekül eine kationische Gruppe, insbesondere eine Ammonium-Gruppe, und eine anionische Gruppe, die eine Carboxylat-Gruppe, Sulfat-Gruppe oder Sulfonat-Gruppe sein kann. Geeignete Betaine sind Alkylbetaine wie Cocobetain oder Fettsäurealkylamidopropylbetaine, beispielsweise Cocosacylamidopropyldimethylbetain, C₁₂-C₁₈-Dimethylaminohexanoate oder C₁₀-C₁₈-Acylamidopropandimethylbetaine.

In einer bevorzugten Ausführungsform der Erfindung enthalten die wässrigen Tensidsysteme ein oder mehrere Amidopropylbetaine der allgemeinen Formel (II), 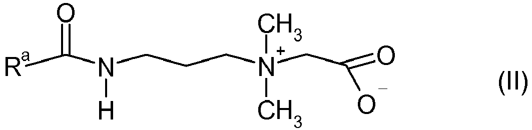 worin R^{a} eine lineare oder verzweigte gesättigte C₇-C₂₁ Alkylgruppe oder eine lineare oder verzweigte ein- oder mehrfach ungesättigte C₇-C₂₁ Alkenylgruppe ist.

In einer weiteren bevorzugten Ausführungsform der Erfindung enthalten die Tensidsysteme ein oder mehrere Betaine der Formel (III), 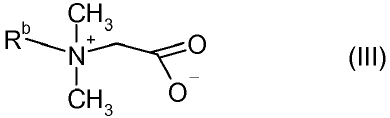 worin R^{b} eine lineare oder verzweigte gesättigte C₈-C₂₂ Alkylgruppe oder eine lineare oder verzweigte ein- oder mehrfach ungesättigte C₈-C₂₂ Alkenylgruppe ist.

In einer weiteren bevorzugten Ausführungsform der Erfindung enthalten die Tensidsysteme ein oder mehrere Sulfobetaine der Formel (IV), 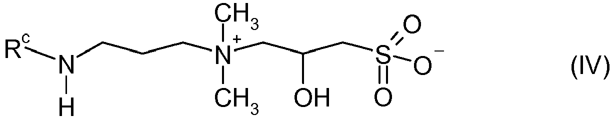 worin R^{c} eine lineare oder verzweigte gesättigte C₈-C₂₂ Alkylgruppe oder eine lineare oder verzweigte ein- oder mehrfach ungesättigte C₈-C₂₂ Alkenylgruppe ist.

Besonders bevorzugt enthalten die Tensidsysteme neben einem oder mehreren Alkylsulfaten und/oder Alkylethersulfaten ein oder mehreren Betaintenside ausgewählt aus der Gruppe der Verbindungen bestehend aus den Amidopropylbetainen der Formel (II), den Betainen der Formel (III) und den Sulfobetainen der Formel (IV).

In einer insbesondere bevorzugten Ausführungsform der Erfindung enthalten die Tensidlösungen ein oder mehrere Betaintenside ausgewählt aus den Amidopropylbetainen der Formel (II).

In einer weiteren insbesondere bevorzugten Ausführungsform der Erfindung enthalten die Tensidlösungen ein oder mehrere Betaintenside ausgewählt aus den Betainen der Formel (III).

In einer weiteren insbesondere bevorzugten Ausführungsform der Erfindung enthalten die Tensidlösungen ein oder mehrere Betaintenside ausgewählt aus den Sulfobetainen der Formel (IV).

Vorzugsweise ist der Rest R^{a} in dem einen oder den mehreren Amidopropylbetainen der Formel (II) eine lineare oder verzweigte gesättigte C₇-C₁₇ Alkylgruppe. Unter den linearen und verzweigten gesättigten Alkylgruppen R^{a} sind die linearen gesättigten Alkylgruppen bevorzugt.

Besonders bevorzugt handelt es sich bei den Amidopropylbetainen der Formel (II) um Cocamidopropylbetaine.

Vorzugsweise ist der Rest R^{b} in dem einen oder den mehreren Betainen der Formel (II) eine lineare oder verzweigte gesättigte C₈-C₁₈-Alkylgruppe und besonders bevorzugt eine lineare oder verzweigte gesättigte C₁₂-C₁₈-Alkylgruppe. Unter den linearen und verzweigten gesättigten Alkylgruppen R^{b} sind die linearen gesättigten Alkylgruppen bevorzugt.

Vorzugsweise ist der Rest R^{c} in der einen oder den mehreren Sulfobetainen der Formel (IV) eine lineare oder verzweigte gesättigte C₈-C₁₈ Alkylgruppe und besonders bevorzugt eine lineare oder verzweigte gesättigte C₁₂-C₁₈ Alkylgruppe. Unter den linearen und verzweigten gesättigten Alkylgruppen R^{c} sind die linearen gesättigten Alkylgruppen bevorzugt.

Besonders bevorzugt enthalten die wässrigen Tensidsysteme Amidopropylbetaine der Formel (I) und/oder Alkylbetaine der Formel (II).

Optionale weitere Tenside (D) können kationische, nichtionische oder amphotere Tenside sein.

Geeignete kationische Tenside sind substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder -bromid, (C₈-C₂₂)-Alkyldimethylbenzylammoniumchlorid oder -bromid, (C₈-C₂₂)-Alkyl-dimethylhydroxyethylammoniumchlorid, -phosphat, -sulfat, -lactat, besonders bevorzugt Distearyldimethylammoniumchlorid, Di(C₈-C₂₂)-Alkylamidopropyltrimethylammoniumchlorid und -methosulfat.

Die Menge der kationischen Tenside in den erfindungsgemäßen Zusammensetzungen kann bis zu 10 Gew.-%, bezogen auf das Gesamtgewicht der fertigen Zusammensetzungen, betragen. Bevorzugt enthalten erfindungsgemäße Zusammensetzungen keine kationischen Polymere.

Als nichtionische Tenside kommen beispielsweise folgende Verbindungen in Frage:
Polyethylen-, Polypropylen- und Polybutylenoxidkondensate von Alkylphenolen. Diese Verbindungen umfassen die Kondensationsprodukte von Alkylphenolen mit einer C₆- bis C₂₀-Alkylgruppe, die entweder linear oder verzweigt sein kann, mit Alkenoxiden. Diese Tenside werden als Alkylphenolalkoxylate, z.B. Alkylphenolethoxylate, bezeichnet.

Kondensationsprodukte von aliphatischen Alkoholen mit 1 bis 25 mol Ethylenoxid. Die Alkyl- oder Alkenylkette der aliphatischen Alkohole kann linear oder verzweigt, primär oder sekundär sein, und enthält im Allgemeinen 8 bis 22 Kohlenstoffatome. Besonders bevorzugt sind die Kondensationsprodukte von C₁₀- bis C₂₀-Alkoholen mit 2 bis 18 mol Ethylenoxid pro mol Alkohol. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol® 15-S-9 (Kondensationsprodukt eines linearen sekundären C₁₁-C₁₅-Alkohols mit 9 mol Ethylenoxid), Tergitol® 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol®-Marken der Clariant.

Kondensationsprodukte von Ethylenoxid mit einer hydrophoben Basis, gebildet durch Kondensation von Propylenoxid mit Propylenglykol. Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic®-Marken der BASF und die Genapol® PF-Marken der Clariant.

Kondensationsprodukte von Ethylenoxid mit einem Reaktionsprodukt von Propylenoxid und Ethylendiamin. Die hydrophobe Einheit dieser Verbindungen besteht aus dem Reaktionsprodukt von Ethylendiamin mit überschüssigem Propylenoxid und weist im Allgemeinen ein Molekulargewicht von 2500 bis 3000 auf. An diese hydrophobe Einheit wird Ethylenoxid bis zu einem Gehalt von 40 bis 80 Gew.-% Polyoxyethylen und einem Molekulargewicht von 5000 bis 11000 addiert. Kommerziell erhältliche Beispiele dieser Verbindungsklasse sind die Tetronic®-Marken der BASF und die Genapol® PN-Marken der Clariant.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, Alkyloligoglycoside, Alkenyloligoglycoside und Fettsäure-N-alkylglucamide.

Die Menge der nichtionischen Tenside in den erfindungsgemäßen Zusammensetzungen kann bis zu 10 Gew.-%, bezogen auf das Gesamtgewicht der fertigen Zusammensetzungen, betragen.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z.B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Die Menge der amphoteren Tenside in den erfindungsgemäßen Zusammensetzungen kann bis zu 10 Gew.-%, bezogen auf das Gesamtgewicht der fertigen Zusammensetzungen, betragen.

Als Rückfettungsmittel (e) können vorzugsweise Lanolin und Lecithin, nicht ethoxylierte und polyethoxylierte oder acylierte Lanolin- und Lecithinderivate, Polyolfettsäureester, Mono-, Di- und Triglyceride (Glyceryloleat, PEG-7 Glyceryloleat) und/oder Fettsäurealkanolamide (Cocamide MEA, Cocamide DEA, Cocamide MIPA) eingesetzt werden, wobei die letzteren gleichzeitig als Schaumstabilisatoren dienen. Bevorzugt werden sie in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt. Das Rückfettungsmittel (e) ist strukturell von den übrigen Komponenten, insbesondere den N-Methyl-N-acylglucaminen (I), verschieden.

Hilfs- und Zusatzstoffe (g) sind beispielsweise Konservierungsmittel, Duftstoffe und Farbstoffe.

Als Konservierungsmittel eignen sich in betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard® DMDMH).

Die Menge der Konservierungsmittel in den erfindungsgemäßen Zusammensetzungen beträgt im Allgemeinen von 0,1 bis 2,0 Gew.-%, bezogen auf das Gesamtgewicht der fertigen Zusammensetzungen.

Als Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Hydroxycitronellal, Lilial und Bourgeonal, zu den Ketonen z.B. die Ionone, alpha-Isomethylionon und Methylcedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Als Duftstoffe können auch natürliche Riechstoffgemische verwendet werden, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die Menge der Duftstoffe in den erfindungsgemäßen Zusammensetzungen beträgt im Allgemeinen von 0 bis 2 Gew.-%, bezogen auf das Gesamtgewicht der fertigen Zusammensetzungen.

Die in den erfindungsgemäßen Zusammensetzungen enthaltenen Farbstoffe und -pigmente, sowohl organische als auch anorganische Farbstoffe, können aus der entsprechenden Positivliste der Kosmetikverordnung bzw. der EG-Liste kosmetischer Färbemittel ausgewählt werden. Vorteilhaft eingesetzt werden auch Perlglanzpigmente, z.B. Fischsilber (Guanin/ Hypoxanthin-Mischkristalle aus Fischschuppen) und Perlmutt (vermahlene Muschelschalen), monokristalline Perlglanzpigmente wie z.B. Bismuthoxychlorid (BiOCI), Schicht-Substrat Pigmente, z.B. Glimmer/Metalloxid, silberweiße Perlglanzpigmente aus TiO₂, Interferenzpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).
Die Menge der Farbstoffe und Pigmente in den erfindungsgemäßen Zusammensetzungen beträgt im Allgemeinen von 0,01 bis 1,0 Gew.-%, bezogen auf das Gesamtgewicht der fertigen Zusammensetzungen.

Offenbart ist auch ein Handgeschirrspülmittel enthaltend
(a) ein oder mehrere N-Methyl-N-acylglucamine (I), die mindestens 8 Gew.-%, bezogen auf die Gesamtmenge an N-Alkyl-N-acylglucaminen (I), Verbindungen mit einem gesättigten C₁₆-, C₁₈- oder einem ein oder mehrfach ungesättigten C₁₈-Fettsäurerest Ra-CO- enthalten als Komponente (A),
(b) ein oder mehrere anionische Tenside aus der Gruppe der Alkylethersulfate, Alkylsulfate und N-Acylaminosäuretenside als Komponente (B),
(c) gegebenenfalls Betain-Tenside als Komponente (C),
(d) gegebenenfalls weitere Tenside als Komponente (D),
(e) ein oder mehrere Rückfettungsmittel als Komponente (E),
(f) Wasser als Komponente (F) sowie
(g) gegebenenfalls weitere Additive, wie Konservierungsmittel, Duftstoffe und Farbstoffe, als Komponente (G),
wobei das Handgeschirrspülmittel vorzugsweise keine kationischen Polymere enthält. Bevorzugte Komponenten a), b), c), d), e) und g) entsprechen den oben Genannten.

Im Allgemeinen enthalten die offenbarten Handgeschirrspülmittel
(a) 0,1 bis 10,0 Gew.-%, bevorzugt 1 bis 5 Gew.-% der Komponente (A),
(b) 0,1 bis 15 Gew.-%, bevorzugt 1 bis 10 Gew.-% der Komponente (B),
(c) 0 bis 10 Gew.-%, bevorzugt 1 bis 8 Gew.-% der Komponente (C),
(d) 0 bis 10 Gew.-%, bevorzugt 1 bis 6 Gew.-% der Komponente (D),
(e) 0,01 bis 10 Gew.-%, bevorzugt 1 bis 5 Gew.-% der Komponente (E),
(f) 45 bis 99,8 Gew.-%, bevorzugt 75 bis 95 Gew.-% der Komponente (F),
(g) 0 bis 10 Gew.-%, bevorzugt 0,1 bis 5 Gew.-% der Komponente (G).

Vorzugsweise enthalten die offenbarten Handgeschirrspülmittel die oben beschriebenen Alkylsulfate und/oder Alkylethersulfate und Betaintenside.

Die Erfindung wird durch die nachstehenden Beispiele näher erläutert.

### Beispiele

Die im Folgenden beschriebene Glucamide wurde nach EP 0 550 637 aus den korrespondierenden Fettsäuremethylestern und N-Methylglucamin in Gegenwart von 1,2 Propylenglykol als Lösemittel hergestellt und als Feststoff bestehend aus Aktivsubstanz und 1,2 Propylenglykol erhalten (alle Angaben in Gew.-%).

**Tabelle 1: Herstellungsbeispiele für N-Methyl-N-acylglucamine**

| Herstellbeispiel | Methylester | Triglycerid | Aktivsubstanz | 1,2-Propylen glykol | Schmelzpunkt |
|---|---|---|---|---|---|
| | | | (%) | (%) | (°C) |
| 1 | C12/14 (C12: 70 %, C14 30 %) | - | 90 | 10 | 85 |
| 2 | | Cocosöl (C8: 6 %; C 10: 6 %; C12: 48 % C14: 20 % C16: 10 %; C18: 2 %, C18' = 8 %) | 90 | 10 | 50 |
| 3 | C12/18 ungesättigt (C12: 60 %, C14: 26 %, C16: 4 % C18: 1 % C18 (Ölsäure): 8 % C18" = 1 % | - | 90 | 10 | 70 |
| 4 | C16/18 ungesättigt C16: 32 % C18: 8 % C18' = 52 % C18" = 8 % | - | 80 | 20 | 45 |

Es wurden wässerige Tensidsysteme bestehend aus Natrium Laurylethersulfat (SLES) [Ethoxylierungsgrad 2 EO] (Genapol® LRO liq., Clariant), Cocamidopropylbetain (Genagen® CAB 818, Clariant) und Zuckertensiden in den Massenverhältnissen gemäß der folgenden Tabelle hergestellt und durch Zugabe von Kochsalz auf eine einheitliche Viskosität von 5000 mPas angepasst. Der pH-Wert wurde auf pH = 5,5 eingestellt. Der Gesamttensidgehalt betrug jeweils 15 %.

Die resultierenden Tensidsysteme wurden in Hautwaschtests sensorisch bewertet.

**Tabelle 2: Beispiele für Anwendungen/Hautreinigungsmittel**

| Beispiel | Zusammensetzung | Verhältnis | Hautgefühl nass | Hautgefühl trocken |
|---|---|---|---|---|
| Vergleichsbeispiel 1 | SLES/Betaine | 7 : 3 | ○ schlüpfrig | ○ entfettet |
| Vergleichsbeispiel 2 | SLES/Betaine/ Herstellbeispiel 1 | 6 : 3 : 1 | - stumpf | - stark entfettet |
| Beispiel 1 | SLES/Betaine/ Herstellbeispiel 2 | 6 : 3 : 1 | + angenehm | + rückfettend |
| Beispiel 2 | SLES/Betaine/ Herstellbeispiel 3 | 6 : 3 : 1 | + angenehm | + rückfettend |
| Vergleichsbeispiel 3 | SLES/Betaine/ Coco-Glucoside | 6 : 3 : 1 | + angenehm | ○ entfettet |

Wie aus den Beispielen 1 und 2 und den Vergleichsbeispielen 1-3 hervorgeht, verleihen die Glucamide aus Beispiel 1-3 im Gegensatz zum Basissystem (Vergleichsbeispiel 1) und zu einem Glucamid mit C12/14 Kettenschnitt (Vergleichsbeispiel 2) auf der Haut positive sensorische Effekte. Vergleichbare Zuckertenside (Vergleichsbeispiel 3) weisen diesen Effekt ebenfalls nicht auf.

### Formulierungsbeispiel 1 (Vergleichsbeispiel)

**Duschbad**

| | | |
|---|---|---|
| Nartium Laureth Sulfat (2 EO) | 8 | % |
| Cocamidopropyl Betain | 3 | % |
| N-Alkyl-N-acylglucamin nach Herstellbeispiel 2 | 2 | % |
| Cocamide MEA | 0,5 | % |
| Natriumchlorid | 0,5 | % |
| Parfüm | 0,5 | % |
| Konservierungsmittel | q.s. | |
| Wasser | ad 100 % | |

### Formulierungsbeispiel 2 (Vergleichsbeispiel)

**Duschbad**

| | | |
|---|---|---|
| Natrium Laureth Sulfat (2 EO) | 8 | % |
| Cocamidopropyl Betain | 3 | % |
| N-Alkyl-N-acylglucamin nach Herstellbeispiel 4 | 2 | % |
| Cocamid MEA | 0,5 | % |
| Natriumchlorid | 0,5 | % |
| Parfüm | 0,5 | % |
| Polyquaternium-7 | 0,2 | % |
| Konservierungsmittel | q.s. | |
| Wasser | ad 100 % | |

### Formulierungsbeispiel 3

**Handgeschirrspülmittel**

| | | |
|---|---|---|
| Natrium Laureth Sulfat (2 EO) | 8 | % |
| Cocamidopropyl Betain | 3 | % |
| N-Alkyl-N-acylglucamin nach Herstellbeispiel 4 | 2 | % |
| Parfüm | 0,2 | % |
| Konservierungsmittel | q.s. | |
| Wasser | ad 100 % | |

Die genannten Prozentzahlen entsprechen Gew.-% und sind auf den Gehalt an Aktivkomponente bezogen.

### Anwendungsbeispiel (Vergleichsbeispiel)

Eine erfindungsgemäße Duschbadformulierung B wurde bezüglich der Parfümstabilisierung beurteilt. Dazu wurde die Formulierung hergestellt, mit Parfüm versetzt, 2 Wochen bei 40 °C in verschlossenen Glasflaschen gelagert und anschließend von einem Panel von drei trainierten Bewertern auf die verbleibende Parfümintensität im Vergleich zu einer bei 25 °C gelagerten Vergleichsformulierung A bewertet.

| Formulierung | Vergleichsformulierung A Zusammensetzung (Gew.-%) | Formulierung B Zusammensetzung (Gew.-%) |
|---|---|---|
| Natriumlaurylethersulfat | 9 | 7.3 |
| Cocamidopropylbetain | 3 | 2.75 |
| Glucamid nach Herstellbeispiel 3 | 0 | 0.92 |
| PEG-40 hydr. Castoroil | 0.2 | 0 |
| PEG-200 Glyceryl Palmate | 0.5 | 0 |
| PEG-7 Glyceryl Cocoate | 0.5 | 0 |
| Natriumbenzoat | 0.2 | 0.2 |
| Natriumsalicylat | 0.2 | 0.2 |
| Polyquaternium-7 | 0.2 | 0.2 |
| Parfüm "waterlilly" | 0.5 | 0.5 |
| Glycerin | 0.8 | 0.8 |
| Wasser | ad 100 | ad 100 |
| Bewertung des Geruchs nach zwei Wochen Lagerzeit bei 40 °C | Deutlich verringerte Parfümintensität, Kopfnote abgebaut | Parfümintensität weitgehend unverändert |

Das Versuchsergebnis zeigt, dass Formulierung B, in der keine Ethoxylate mit endständigen OH-Gruppen wie PEG-40 hydr. Castoroil, PEG-200 Glyceryl Palmate oder PEG-7 Glyceryl Cocoate enthalten sind, dafür aber ein Glucamid, eine deutlich bessere Parfümstabilität bei Warmlagerung aufweist. Es kann also geschlossen werden, dass die Verwendung von Glucamiden anstelle von Ethoxylaten mit endständigen OH-Gruppen als hautpflegende Komponente, als Solubilisator oder Verdickungsmittel gleichzeitig zu einer Verbesserung der Parfümstabilität führt.

## Patentansprüche

1. Verwendung von N-Alkyl-N-acylglucaminen der Formel (I) in Handgeschirrspülmitteln, die ein wässriges Tensidsystem, enthaltend mindestens ein anionisches Tensid, umfassen, 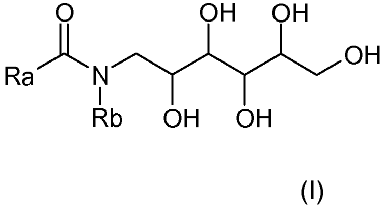 wobei in der Formel (I)
Ra einen linearen oder verzweigten, gesättigten oder ungesättigten C₅-C₂₁-Alkylrest und
Rb einen C₁-C₄ Alkylrest bedeutet und
wobei die N-Alkyl-N-acylglucamine (I) mindestens 8 Gew.-%, bezogen auf die Gesamtmenge an N-Alkyl-N-acylglucaminen (I), Verbindungen mit einem ein oder mehrfach ungesättigten C₁₈-Fettsäurerest Ra-CO- enthalten.

2. Verwendung nach Anspruch 1, wobei Rb einen Methylrest bedeutet.

3. Verwendung nach Anspruch 1 oder 2, wobei der Rest R^{a} von Ölsäure, Linolsäure oder Linolensäure abgeleitet ist.

4. Verwendung nach einem der Ansprüche 1 bis 3, wobei die N-Alkyl-N-acylglucamine (I) mindestens 15 Gew.-% N-Alkyl-N-acylglucamine mit einem ein oder mehrfach ungesättigten C₁₈- Fettsäurerest enthalten.

5. Verwendung nach einem der Ansprüche 1 bis 4, wobei die N-Alkyl-N-acylglucamine (I) mindestens 8 Gew.-% N-Alkyl-N-acylglucamine mit einem einfach ungesättigten C₁₈- Fettsäurerest enthalten.

6. Verwendung nach einem der Ansprüche 1 bis 5, wobei die N-Alkyl-N-acylglucamine (I) mindestens 8 Gew.-% N-Alkyl-N-acylglucamine mit einem ungesättigten C₁₈- Fettsäurerest enthalten und mindestens 30 Gew.-% mit einem gesättigten C₁₂- Fettsäurerest enthalten.

7. Verwendung nach einem der Ansprüche 1 bis 6, **dadurch gekennzeichnet, dass** die wässrigen Tensidsysteme ein Alkylsulfat und/oder ein Alkylethersulfat als anionisches Tensid enthalten.

8. Verwendung nach einem der Ansprüche 1 bis 7, **dadurch gekennzeichnet, dass** die wässrigen Tensidsysteme ein Alkylsulfat und/oder ein Alkylethersulfat als anionisches Tensid und ein Betaintensid enthalten.

9. Verwendung nach einem der Ansprüche 1 bis 8, **dadurch gekennzeichnet, dass** die wässrigen Tensidsysteme ein lineares C₈-C₂₀-Alkylsulfat und/oder ein lineares C₈-C₂₀-Alkylethersulfat enthalten.

10. Verwendung nach Anspruch 9, **dadurch gekennzeichnet, dass** die wässrigen Tensidsysteme Laurylsulfat und/oder ein Laurylethersulfat enthalten.

11. Verwendung nach einem der Ansprüche 1 bis 10, **dadurch gekennzeichnet, dass** die wässrigen Tensidsysteme ein Acylamidopropylbetain oder ein Alkylbetain enthält.

12. Handgeschirrspülmittel, enthaltend
(a) ein oder mehrere N-Methyl-N-acylglucamine (I), die mindestens 8 Gew.-%, bezogen auf die Gesamtmenge an N-Alkyl-N-acylglucaminen (I), Verbindungen mit einem gesättigten C₁₆-, C₁₈- oder einem ein oder mehrfach ungesättigten C₁₈-Fettsäurerest Ra-CO- enthalten als Komponente (A),
(b) ein oder mehrere anionische Tenside aus der Gruppe der Alkylethersulfate, Alkylsulfate und N-Acylaminosäuretenside als Komponente (B),
(c) gegebenenfalls Betain-Tenside als Komponente (C),
(d) gegebenenfalls weitere Tenside als Komponente (D),
(e) ein oder mehrere Rückfettungsmittel als Komponente (E),
(f) Wasser als Komponente (F) sowie
(g) gegebenenfalls weitere Additive, wie Konservierungsmittel, Duftstoffe und Farbstoffe, als Komponente (G).

13. Handgeschirrspülmittel gemäß Anspruch 12 mit der Maßgabe, dass es keine kationischen Polymere enthält.

14. Handgeschirrspülmittel gemäß Anspruch 12 oder 14, enthaltend
(a) 0,1 bis 10,0 Gew.-% der Komponente (A),
(b) 0,1 bis 15 Gew.-% der Komponente (B),
(c) 0 bis 10 Gew.-% der Komponente (C),
(d) 0 bis 10 Gew.-% der Komponente (D),
(e) 0,01 bis 10 Gew.-% Gew.-% der Komponente (E),
(f) 45 bis 99,8 Gew.-% der Komponente (F),
(g) 0 bis 10 Gew.-% der Komponente (G).

## Claims

1. The use of N-alkyl-N-acylglucamines of the formula (I) in hand dishwashing compositions which comprise an aqueous surfactant system comprising at least one anionic surfactant, 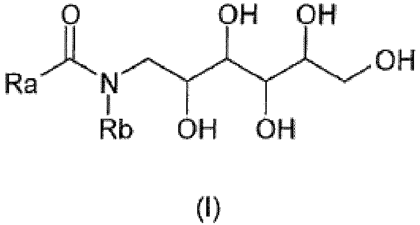 where in the formula (I)
Ra is a linear or branched, saturated or unsaturated C₅-C₂₁ alkyl radical and
Rb is a C₁-C₄ alkyl radical,
the N-alkyl-N-acylglucamines (I) comprising at least 8 wt%, based on the total amount of N-alkyl-N-acylglucamines (I), of compounds having a singly or multiply unsaturated C₁₈ fatty acid radical Ra-CO-.

2. Use according to Claim 1, Rb being a methyl radical.

3. Use according to Claim 1 or 2, the radical R^{a} deriving from oleic acid, linoleic acid, or linolenic acid.

4. Use according to any of Claims 1 to 3, the N-alkyl-N-acylglucamines (I) comprising at least 15 wt% of N-alkyl-N-acylglucamines having a singly or multiply unsaturated C₁₈ fatty acid radical.

5. Use according to any of Claims 1 to 4, the N-alkyl-N-acylglucamines (I) comprising at least 8 wt% of N-alkyl-N-acylglucamines having a singly unsaturated C₁₈ fatty acid radical.

6. Use according to any of Claims 1 to 5, the N-alkyl-N-acylglucamines (I) comprising at least 8 wt% of N-alkyl-N-acylglucamines having an unsaturated C₁₈ fatty acid radical and at least 30 wt% having a saturated C₁₂ fatty acid radical.

7. Use according to any of Claims 1 to 6, wherein the aqueous surfactant systems comprise an alkyl sulfate and/or an alkyl ether sulfate as anionic surfactant.

8. Use according to any of Claims 1 to 7, wherein the aqueous surfactant systems comprise an alkyl sulfate and/or an alkyl ether sulfate as anionic surfactant and a betaine surfactant.

9. Use according to any of Claims 1 to 8, wherein the aqueous surfactant systems comprise a linear C₈-C₂₀ alkyl sulfate and/or a linear C₈-C₂₀ alkyl ether sulfate.

10. Use according to Claim 9, wherein the aqueous surfactant systems comprise lauryl sulfate and/or a lauryl ether sulfate.

11. Use according to any of Claims 1 to 10, wherein the aqueous surfactant systems comprise an acylamidopropylbetaine or an alkylbetaine.

12. Hand dishwashing composition comprising
(a) one or more N-methyl-N-acylglucamines (I) containing at least 8 wt%, based on the total amount of N-alkyl-N-acylglucamines (I), of compounds having a saturated C₁₆, C₁₈, or singly or multiply unsaturated C₁₈ fatty acid radical Ra-CO-, as component (A),
(b) one or more anionic surfactants from the group of the alkyl ether sulfates, alkyl sulfates, and N-acylamino acid surfactants as component (B),
(c) optionally betaine surfactants as component (C),
(d) optionally further surfactants as component (D),
(e) one or more refatting agents as component (E),
(f) water as component (F), and
(g) optionally further additives, such as preservatives, fragrances, and dyes, as component (G).

13. Hand dishwashing composition according to Claim 12, with the proviso that it contains no cationic polymers.

14. Hand dishwashing composition according to Claim 12 or 14 comprising
(a) 0.1 to 10.0 wt% of component (A),
(b) 0.1 to 15 wt% of component (B),
(c) 0 to 10 wt% of component (C),
(d) 0 to 10 wt% of component (D),
(e) 0.01 to 10 wt% wt% of component (E),
(f) 45 to 99.8 wt% of component (F),
(g) 0 to 10 wt% of component (G).

## Revendications

1. Utilisation de N-alkyl-N-acylglucamines de formule (I) dans des agents de lavage de la vaisselle à la main, qui comprennent un système de tensioactifs aqueux, contenant au moins un tensioactif anionique, 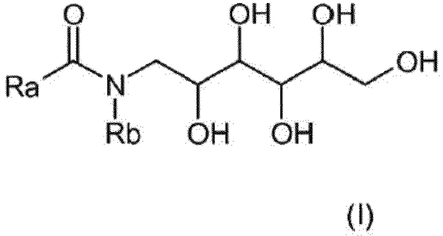 dans la formule (I),
Ra signifiant un radical alkyle en C₅-C₂₁ linéaire ou ramifié, saturé ou insaturé, et
Rb signifiant un radical alkyle en C₁-C₄, et
les N-alkyl-N-acylglucamines (I) contenant au moins 8 % en poids, par rapport à la quantité totale de N-alkyl-N-acylglucamines (I), de composés contenant un radical acide gras en C₁₈ mono- ou polyinsaturé Ra-CO-.

2. Utilisation selon la revendication 1, dans laquelle Rb signifie un radical méthyle.

3. Utilisation selon la revendication 1 ou 2, dans laquelle le radical Ra est dérivé d'acide oléique, d'acide linoléique ou d'acide linolénique.

4. Utilisation selon l'une quelconque des revendications 1 à 3, dans laquelle les N-alkyl-N-acylglucamines (I) contiennent au moins 15 % en poids de N-alkyl-N-acylglucamines contenant un radical acide gras en C₁₈ mono- ou polyinsaturé.

5. Utilisation selon l'une quelconque des revendications 1 à 4, dans laquelle les N-alkyl-N-acylglucamines (I) contiennent au moins 8 % en poids de N-alkyl-N-acylglucamines contenant un radical acide gras en C₁₈ monoinsaturé.

6. Utilisation selon l'une quelconque des revendications 1 à 5, dans laquelle les N-alkyl-N-acylglucamines (I) contiennent au moins 8 % en poids de N-alkyl-N-acylglucamines contenant un radical acide gras en C₁₈ insaturé et au moins 30 % en poids contenant un radical acide gras en C₁₂ saturé.

7. Utilisation selon l'une quelconque des revendications 1 à 6, **caractérisée en ce que** les systèmes de tensioactifs aqueux contiennent un alkylsulfate et/ou un alkyléthersulfate en tant que tensioactif anionique.

8. Utilisation selon l'une quelconque des revendications 1 à 7, **caractérisée en ce que** les systèmes de tensioactifs aqueux contiennent un alkylsulfate et/ou un alkyléthersulfate en tant que tensioactif anionique et un tensioactif à base de bétaïne.

9. Utilisation selon l'une quelconque des revendications 1 à 8, **caractérisée en ce que** les systèmes de tensioactifs aqueux contiennent un alkylsulfate en C₈-C₂₀ linéaire et/ou un alkyléthersulfate en C₈-C₂₀ linéaire.

10. Utilisation selon la revendication 9, **caractérisée en ce que** les systèmes de tensioactifs aqueux contiennent du laurylsulfate et/ou un lauryléthersulfate.

11. Utilisation selon l'une quelconque des revendications 1 à 10, **caractérisée en ce que** les systèmes de tensioactifs aqueux contiennent une acylamidopropylbétaïne ou une alkylbétaïne.

12. Agent de lavage de la vaisselle à la main, contenant :
(a) une ou plusieurs N-méthyl-N-acylglucamines (I), qui contiennent au moins 8 % en poids, par rapport à la quantité totale de N-alkyl-N-acylglucamines (I), de composés contenant un radical acide gras en C₁₆, C₁₈ saturé ou en C₁₈ mono- ou polyinsaturé Ra-CO- en tant que composant (A),
(b) un ou plusieurs tensioactifs anioniques du groupe des alkyléthersulfates, des alkylsulfates et des tensioactifs à base d'acides N-acylaminés en tant que composant (B),
(c) éventuellement des tensioactifs à base de bétaïne en tant que composant (C),
(d) éventuellement d'autres tensioactifs en tant que composant (D),
(e) un ou plusieurs agents surgraissants en tant que composant (E),
(f) de l'eau en tant que composant (F), et
(g) éventuellement d'autres additifs, tels que des conservateurs, des parfums et des colorants, en tant que composant (G).

13. Agent de lavage de la vaisselle à la main selon la revendication 12, à condition qu'il ne contienne pas de polymères cationiques.

14. Agent de lavage de la vaisselle à la main selon la revendication 12 ou 14, contenant :
(a) 0,1 à 10,0 % en poids du composant (A),
(b) 0,1 à 15 % en poids du composant (B),
(c) 0 à 10 % en poids du composant (C),
(d) 0 à 10 % en poids du composant (D),
(e) 0,01 à 10 % en poids % en poids du composant (E),
(f) 45 à 99,8 % en poids du composant (F),
(g) 0 à 10 % en poids du composant (G).
